# EUROPEAN PATENT APPLICATION

(11) **EP 4 376 018 A1**
(43) Date of publication of application: **29.05.2024**
(21) Application number: 23212368.7
(22) Date of filing: 27.11.2023
(51) Int. Cl.: G16H 30/20, G16H 30/40, G16H 40/67

(54) **MEDICAL INFORMATION PROCESSING APPARATUS, MEDICAL INFORMATION PROCESSING METHOD, AND COMPUTER PROGRAM**

(30) Priority: 28.11.2022 JP 2022188827
(71) Applicant: CANON KABUSHIKI KAISHA, Tokyo 146-8501 (JP)
(72) Inventor: ARIMA, Keisuke, Tokyo, 146-8501 (JP)
(74) Representative: Canon Europe Limited

(57) **Abstract**

A medical information processing apparatus includes an obtaining means that obtains a processing result of an original image that is a medical image and is obtained through processing performed in an external processing apparatus, and an output means that outputs the processing result to an image management server, based on a setting as to whether to enable output of the processing result to the image management server. In a case where an examination including capturing of the original image is not ended by an obtained timing of the processing result, the output means outputs the processing result to the image management server in response to an end of the examination. In a case where the examination is ended by the obtained timing of the processing result, the output means outputs the processing result to the image management server at the obtained timing of the processing result.

## Description

### FIELD OF THE INVENTION

The present disclosure relates to a medical information processing apparatus, a medical information processing method, and a computer program.

### BACKGROUND

Description of the Related Art: In the related art, there is a radiographing system including a radiation detection apparatus that irradiates a subject with radiation (e.g., X-rays) and detects an intensity distribution of the radiation transmitted through the subject to capture a radiographic image of a target.

In general, for an examination using radiation (e.g., radiation examination), a doctor in each clinical department sets examination information that includes an imaging-target portion and an imaging method. Radiographic imaging is then performed based on the set examination information by using the radiographing system.

In recent years, progress has been made with the use of artificial intelligence (Al) technology for assisting diagnosis based on medical images. The radiographing system has image processing installed therein for assisting with such diagnosis. However, the use of image processing outside the radiographing system can provide more useful assistance to doctors.

The radiographing system cooperates with an external image processing apparatus to transmit an image serving as a processing target and receive an image resulting from the processing. In this way an image can be provided, for diagnosis, that has been subjected to processing which is not installed in the radiographing system. Japanese Patent Laid-Open No. 2004-295184 discloses a configuration in which a medical image is transmitted to a processing server and computer-aided-diagnosis (CAD) processing is performed.

### SUMMARY OF THE INVENTION

The present disclosure in its first aspect provides a medical information processing apparatus comprising an obtaining means configured to obtain a processing result of an original image that is a medical image, the processing result being obtained through processing performed in an external processing apparatus; and an output means configured to output the processing result to an image management server, based on a setting as to whether to enable output of the processing result to the image management server. The output means is configured, in a case where an examination including capturing of the original image is not ended by an obtained timing of the processing result, to output the processing result to the image management server in response to an end of the examination. The output means is configured, in a case where the examination is ended by the obtained timing of the processing result, to output the processing result to the image management server at the obtained timing of the processing result.
Optional features of the disclosure are outlined in the following passages.
The processing result may include an image generated from the original image through (i.e., as a result of) the processing performed by the external processing apparatus.
The obtaining means may be configured to treat the processing result as being rejected in a case where the original image is determined to be a rejected image.
The output means may be configured to not output the processing result to the image management server in a case where the original image is determined to be a rejected image.
The apparatus may comprise a display control means configured to display a thumbnail of the original image and a thumbnail of a processed image included in the processing result on a display unit. The display control means may be configured to display the thumbnail of the processed image in a different display style from the thumbnail of the original image to make the processed image identifiable as an image that has been subjected to the processing in the external processing apparatus.
The apparatus may comprise a display control means configured to display a thumbnail of a processed image included in the processing result on a display unit. The display control means may be configured to display at least one of the following:
an object indicating that the processed image is an image that has been subjected to the processing in the external processing apparatus;
the thumbnail of the processed image and the object in at least one (e.g., any) of a superimposed display style, a parallel display style, and a composite display style;
an object indicating that the original image is an image to be subjected to the processing in the external processing apparatus; and
the thumbnail of the original image and the object in at least one (e.g., any) of a superimposed display style, a parallel display style, and a composite display style.
The apparatus may comprise an accepting means configured to accept an image editing instruction for a medical image from an operator. The accepting means may be configured to restrict an image editing instruction for a processed image included in the processing result. Alternatively, or additionally, the accepting means may be configured to request display control means to display a warning in response to accepting an image editing instruction for the processed image.
The apparatus may comprise a transmission means configured to transmit the original image to the external processing apparatus. The apparatus may comprise a setting means configured to make a setting as to whetherto enable transmission of a medical image to the external processing apparatus. The transmission means may be configured to transmit the original image to the external processing apparatus in a case where transmission of a medical image is enabled by the setting means.
The output means may be configured, in a case where the examination including capturing of the original image is displayed on a display unit when a processed image included in the processing result is obtained by the obtaining means, to output the processed image to the image management server in response to an output instruction. The output means may be configured, in a case where the examination is not displayed on the display unit when the processed image is obtained by the obtaining means, to output the processed image to the image management server at an obtained timing of the processed image by the obtaining means.

The present disclosure in its second aspect provides a medical information processing method as specified in claim 11.

The present disclosure in its third aspect provides a computer program as specified in claim 12.

Further features of the present invention will become apparent from the following description of embodiments with reference to the attached drawings.

### BRIEF DESCRIPTION OF THE DRAWINGS

Fig. 1 is a diagram illustrating an example of a schematic configuration of a radiographing system including a medical information processing apparatus according to an embodiment.
Fig. 2 is a diagram illustrating an example of a schematic configuration of a control unit according to the embodiment.
Fig. 3 is a diagram illustrating an example of an imaging method table and an external processing table stored in a storage unit according to the embodiment.
Fig. 4 is a diagram illustrating an example of an image table stored in the storage unit according to the embodiment.
Figs. 5A to 5C are diagrams each illustrating an example of a new examination input screen displayed on a display unit according to the embodiment.
Fig. 6 is a diagram illustrating an example of an imaging screen displayed on the display unit according to the embodiment.
Fig. 7 is a flowchart illustrating an example of a procedure of a processed image registration process, as a medical information processing method performed by the medical information processing apparatus according to the embodiment.
Fig. 8 is a flowchart illustrating an example of a procedure of a processed image display process, as a medical information processing method performed by the medical information processing apparatus according to the embodiment.
Fig. 9 is a flowchart illustrating an example of a procedure of an output process of outputting an image to an image management server, as a medical information processing method performed by the medical information processing apparatus according to the embodiment.
Fig. 10 is a diagram illustrating an example of an examination table and a series table stored in the storage unit according to the embodiment.

### DESCRIPTION OF THE EMBODIMENTS

Embodiments of a radiographing system disclosed in this specification will be described in detail below with reference to the accompanying drawings. Note that components described in these embodiments are merely examples, and the technical scope of the radiographing system disclosed in this specification is defined by the claims and is not limited by the individual embodiments below. In addition, the disclosure of this specification is not limited to the embodiments below, and various variations (including organic combinations of the individual embodiments) can be made based on the gist of the disclosure of this specification. These variations are not excluded from the scope of the disclosure of this specification. That is, all configurations obtained by combining the individual embodiments described below and variations thereof are also included in the embodiments disclosed in this specification. Although a radiographing system will be described below as an example, a modality that images a subject may be another medical imaging apparatus such as a magnetic resonance imaging (MRI) apparatus, an X-ray computed tomography (CT) apparatus, a three-dimensional (3D) ultrasonic imaging apparatus, a photoacoustic tomographic apparatus, a positron emission tomography/single photon emission computed tomography (PET/SPECT) apparatus, or an optical coherence tomography (OCT) apparatus. That is, a series of processes according to the present embodiments can be implemented by a radiographing system including various medical imaging apparatuses and a medical information processing apparatus, and may be applied to not only radiographic images but also other medical images. In addition, the images in the present disclosure include not only images displayed on a display unit but also images stored in a database or a storage unit as image data and images transmitted and received as image data.

### Configuration of Radiographing System

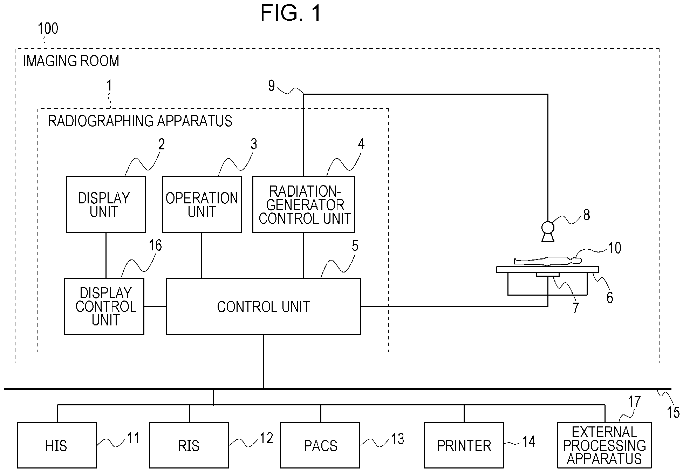

An embodiment will be described with reference to Fig. 1. Fig. 1 is a diagram illustrating an example of a configuration of a radiographing system according to the present embodiment. As illustrated in Fig. 1, the radiographing system according to the present embodiment includes a radiographing apparatus 1 and a hospital information system (HIS) 11 that manages the progress of an examination.

The radiographing system according to the present embodiment also includes a radiology information system (RIS) 12 that delivers an examination order to the radiographing apparatus 1.

The radiographing system according to the present embodiment is connected to a picture archiving and communication system (PACS) 13 that manages a radiographic image, a printer 14 that prints out a radiographic image, and an external processing apparatus 17 (e.g., an external image processing apparatus) that performs processing on a radiographic image.

The HIS 11 is a hospital management system and includes a server that manages accounting information. When performing radiographic imaging, an operator inputs an examination instruction from a terminal (e.g., input unit) of the HIS 11.

The HIS 11 then delivers request information to a radiology department, which is a destination of a request for radiographic imaging, in the hospital. This request information is referred to as an examination order. The examination order includes information such as a name of a requestor department, an examination identifier (ID), an examination item, and patient information (e.g., subject information) on a patient (e.g., subject).

In response to the RIS 12 receiving the examination order, the radiology department adds, as an imaging protocol, imaging information related to radiographic imaging (such as imaging-target portion information, imaging direction information, and technique information) to the examination order, and delivers the resulting examination order to the radiographing apparatus 1. The radiographing apparatus 1 performs radiographic imaging in accordance with the received examination order. The radiographing apparatus 1 obtains a captured radiographic image, generates examination information in which the radiographic image and the examination order are associated with each other, and outputs the examination information together with the radiographic image.

The PACS 13 is a server intended mainly for image management. With a high-definition monitor connected to the PACS 13, radiographic image examination work, detailed post-processing, and diagnostic work are performed. As described above, the radiographic image obtained by the radiographing apparatus 1 is delivered to the PACS 13.

Execution information (such as an image ID and an imaging date and time) of the examination performed in the radiographing apparatus 1 is delivered to the HIS 11. The execution information delivered to the HIS 11 is used in an accounting process following the examination as well as management of the progress of the examination.

The external processing apparatus 17 is an apparatus that performs image processing and the like on a radiographic image. The external processing apparatus 17 performs a diagnosis assistance process using an Al technology, and delivers a generated processed image to an apparatus such as the radiographing apparatus 1 or the PACS 13. The radiographing apparatus 1 presents the processed image received from the external processing apparatus 17 together with a captured image (e.g., an original image to be subjected to processing in the external processing apparatus 17). That is, the external processing apparatus 17 provides a process that is not installed in the radiographing apparatus 1.

The radiographing apparatus 1, the HIS 11, the RIS 12, the PACS 13, the printer 14, and the external processing apparatus 17 are connected to one another via a network 15, which includes a local area network (LAN) or a wide area network (WAN), for example.

Each of these apparatuses includes one or more computers. The one or more computers each include, for example, main control means such as a central processing unit (CPU), and storage means such as a read-only memory (ROM) and a random access memory (RAM). The one or more computers each may also include communication means such as a network card, and input/output means such as a keyboard, a display, and a touch panel. These constituent means are electrically connected to one another by a bus or the like, and are controlled by the main control means executing a program stored in the storage means.

As illustrated in Fig. 1, the radiographing apparatus 1 that performs radiographic imaging is installed in an imaging room 100. In the imaging room 100, a radiation-generator control unit 4 that causes radiation to be generated, a radiation detector 7 that detects radiation transmitted through a subject 10 to capture a radiographic image, and an imaging table 6 are installed.

The radiographing apparatus 1 includes a display unit 2 that displays a radiographic image and various kinds of information, an operation unit 3 that is operated by an operator, and a control unit 5 that controls each component.

The radiation-generator control unit 4 sets imaging conditions related to radiation in a radiation generator 8, and controls the radiation generator 8. The radiation generator 8 functions as a radiation source that generates radiation. The radiation generator 8 is implemented by, for example, a radiation tube, and irradiates the subject 10 (for example, a specific portion of the subject 10) with radiation.

The radiation generator 8 can irradiate a desired irradiation range with radiation. The radiation generator 8 has, at a radiation surface, a diaphragm (not illustrated) that shields radiation. An operator controls the diaphragm that shields radiation and thus can adjust the irradiation range irradiated with radiation by the radiation generator 8.

The radiographing system includes the radiation detector 7 that detects radiation emitted from the radiation generator 8. The radiation detector 7 detects radiation transmitted through the subject 10, and outputs image data according to the radiation. Note that the image data is also referred to as a radiographic image.

Specifically, the radiation detector 7 detects radiation transmitted through the subject 10, as electric charge equivalent to a dose of transmitted radiation. Examples of the radiation detector 7 to be used include a direct conversion sensor that directly converts radiation into electric charge such as a-Se that converts radiation into electric charge and an indirect conversion sensor that uses a scintillator such as Csl and a photoelectric conversion element such as a-Si.

The radiation detector 7 performs analog-to-digital conversion (A/D conversion) on the detected electric charge to generate image data, and accumulates the image data in a storage unit (not illustrated). The radiation detector 7 can assign image information (such as an image ID, an imaging date and time, and a transfer status of image data) to the image data, and transfer the image information to the radiographing apparatus 1 together with the image data.

The display unit 2 is implemented by, for example, a liquid crystal display, and displays various kinds of information to an operator (such as an imaging technician or a doctor, for example). The operation unit 3 includes, for example, a mouse and operation buttons, and inputs to each component various instructions given from the operator. Note that the display unit 2 and the operation unit 3 may be implemented as a touch panel in which the display unit 2 and the operation unit 3 are integrated together.

The control unit 5 of the radiographing apparatus 1 is connected to the radiation detector 7 via a wireless LAN. Image data, a control signal, and the like are transmitted and received between the control unit 5 and the radiation detector 7. That is, the image data stored in the radiation detector 7 as a result of radiographic imaging is output (e.g., transferred) to the control unit 5 via the wireless LAN.

### Description of Radiographing System

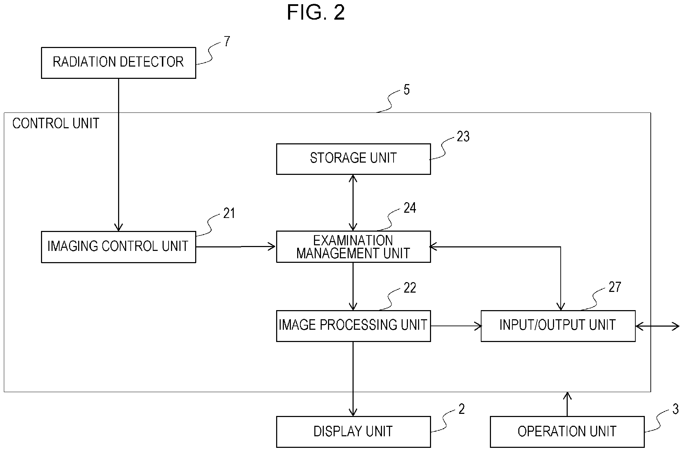

The radiographing system according to the present embodiment will be described in detail with reference to Fig. 2. The radiographing apparatus 1 includes the control unit 5 that performs image processing on a radiographic image output from the radiation detector 7 to generate an image. The control unit 5 has an application function that operates on a computer. The control unit 5 controls the operation of the radiation detector 7, outputs a radiographic image to the display unit 2, and outputs a graphical user interface (GUI). That is, the control unit 5 corresponds to an example of display control means configured to display a thumbnail of a medical image and a thumbnail of a processed image on a display unit. The control unit 5 also functions as accepting means configured to accept an image editing instruction to edit a medical image displayed on the display unit, and can perform image editing such as clipping and annotation based on the instruction from an operator.

The control unit 5 includes an imaging control unit 21 that controls imaging performed by the radiation detector 7, an image processing unit 22 that performs image processing on a radiographic image obtained through imaging, and a storage unit 23 that stores the radiographic image output from the radiation detector 7 and various kinds of information such as an examination order, an imaging protocol, and an imaging method. The control unit 5 also includes an examination management unit 24 that manages examination information in which a radiographic image and an examination order are associated with each other, and an input/output unit 27 that outputs a generated image object to the outside or receives a generated image object input from the outside.

The storage unit 23 stores the examination information managed by the examination management unit 24, the imaging protocol, the imaging method, the radiographic image output from the radiation detector 7, and various kinds of information for use in examination management. The storage unit 23 also stores the imaging protocol associated with the examination order, together with identification information for identifying the imaging protocol.

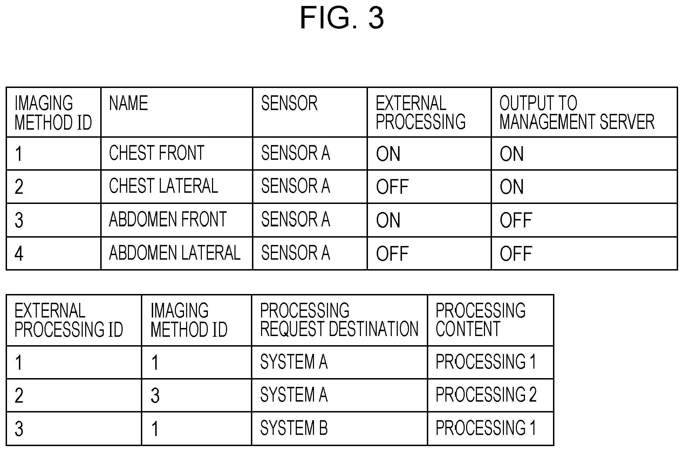

Fig. 3 is a diagram illustrating an example of an imaging method table and an external processing table stored in the storage unit 23 illustrated in Fig. 2. The imaging method table illustrated in Fig. 3 is a table that assigns an imaging method ID to each imaging method and stores setting values of a name, whether external processing is enabled, and so on.

The external processing table is a table that assigns an external processing ID and associates an external processing ID with an imaging method identified by an imaging method ID.

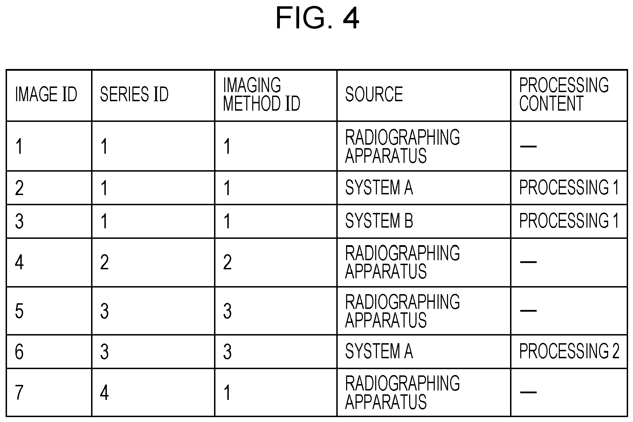

Fig. 4 is a diagram illustrating an example of an image table that stores a captured image, a processed image generated by the external processing apparatus 17, and image information of the captured image or the processed image. The image table stores an ID for each image, and stores a source of the image and, in the case of a processed image, content of processing that has been performed by the external processing apparatus 17, together with a series ID and an imaging method ID.

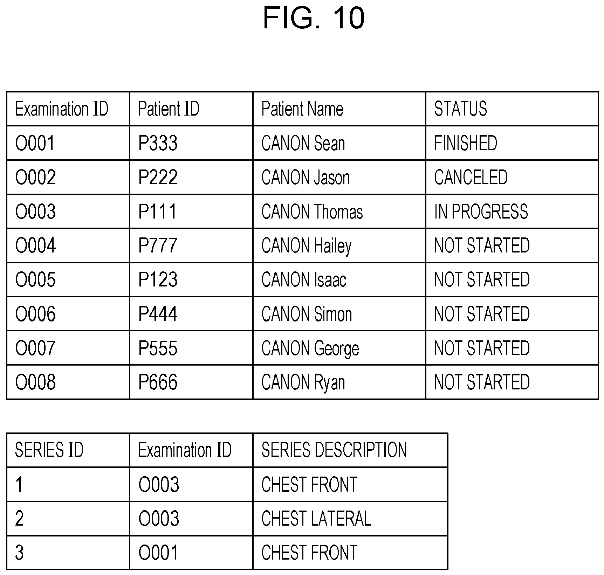

Fig. 10 is a diagram illustrating an example of an examination table and a series table stored in the storage unit 23 illustrated in Fig. 2. The examination table illustrated in Fig. 10 is a table that assigns an examination ID to each examination and stores patient information and a status of the examination. The series table illustrated in Fig. 10 is a table that assigns a series ID to each series and stores series information together with an examination ID.

The examination management unit 24 manages an imaging protocol that is associated with an examination order and that defines an imaging method, imaging conditions, an image processing condition, and so on. For example, when the radiographing apparatus 1 creates examination information, the examination management unit 24 can associate subject information input through the operation unit 3 with the imaging protocol to create new examination information. On the other hand, in a case where an examination is requested from the RIS 12, the examination management unit 24 extracts an imaging protocol stored in the storage unit 23 by using identification information of the imaging protocol associated with the received examination order. The examination management unit 24 associates the extracted imaging protocol with the examination order to create new examination information. The newly created examination information is stored in the storage unit 23. The examination management unit 24 also associates a captured image and a processed image with each other by using accompanying information that accompanies the processed image and image information of the captured image. For example, the examination management unit 24 associates a captured image and a processed image with each other by further adding image information of the captured image, which is an original of the processed image, to the processed image or by rewriting the accompanying information of the processed image to the image information of the captured image. That is, the examination management unit 24 may compare the image information of the captured image with the accompanying information of the processed image. When the accompanying information lacks information compared to the image information, the examination management unit 24 may supplement the lacking information by using the image information or rewrite the accompanying information to the image information. The created information is stored in the storage unit 23.

The imaging control unit 21 transmits, to the radiation detector 7, a transfer request signal for requesting transfer of a radiographic image accumulated in the radiation detector 7, and receives the radiographic image from the radiation detector 7. The imaging control unit 21 manages the received radiographic image together with radiation detector information on the radiation detector 7. The imaging control unit 21 also associates the radiographic image with examination information and the imaging protocol managed by the examination management unit 24.

The image processing unit 22 performs image processing on a radiographic image by using an imaging protocol and image information obtained from the imaging control unit 21. The radiographic image on which the image processing unit 22 has performed image processing is displayed on the display unit 2. Alternatively, the radiographic image on which the image processing unit 22 has performed image processing is output to the outside through the input/output unit 27. The image processing unit 22 performs image processing for adjusting an image such as brightness and/or contrast. The image processing unit 22 can also perform processing such as clipping and annotation on the adjusted radiographic image.

The input/output unit 27 accepts an examination order input from the RIS 12 and a processed image input from the external processing apparatus 17. The input/output unit 27 outputs a radiographic image to an external apparatus such as the PACS 13, the printer 14, or the external processing apparatus 17, and outputs examination execution information to the HIS 11. That is, the input/output unit 27 corresponds to an example of transmission means configured to transmit an original image serving as a processing target to the external processing apparatus 17.

An example of the configuration of the radiographing system according to the present embodiment has been described above. Note that the configuration illustrated in Fig. 1 is merely an example and can be changed as appropriate. For example, in Fig. 1, various apparatuses are connected to the radiographing apparatus 1 via the network 15 but the radiographing apparatus 1 need not be connected to such apparatuses. An image used for diagnosis may be output to a portable medium such as a digital versatile disc (DVD) and may be input to the various apparatuses via the portable medium. In addition, the network 15 may be configured in a wired manner, or a part thereof may be configured by a wireless signal transmission path.

### Imaging Process

A procedure of an imaging process of capturing a radiographic image will be described in accordance with a procedure of an examination performed by the radiographing system illustrated in Fig. 1.

First, the radiographing apparatus 1 receives patient information and examination information, based on an examination request form or an examination request from the RIS 12. The patient information includes a patient name and a patient ID. The examination information includes imaging information that defines content of imaging to be performed on the patient.

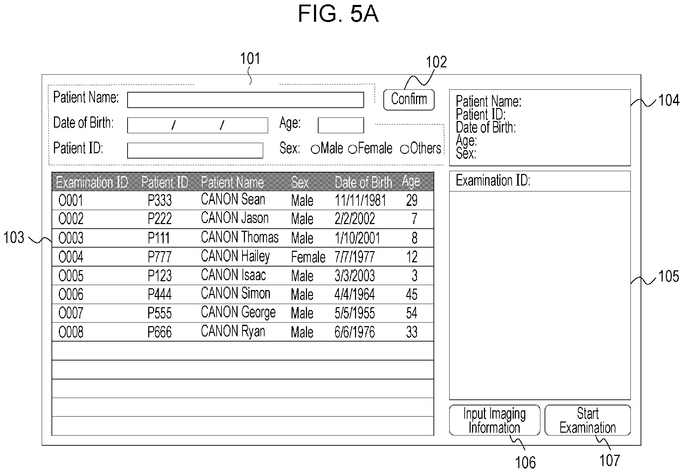

Under the control of a display control unit 16, the radiographing apparatus 1 displays a new examination input screen illustrated in Figs. 5A to 5C on the display unit 2. As illustrated in Fig. 5A, the new examination input screen includes a patient information input area 101, a patient information confirmation button 102, and a requested examination list 103. The new examination input screen also includes a patient information display area 104, an imaging information display area 105, an imaging information input button 106, and an examination start button 107.

The requested examination list 103 displays a list of examinations for which the requests are received from the RIS 12.

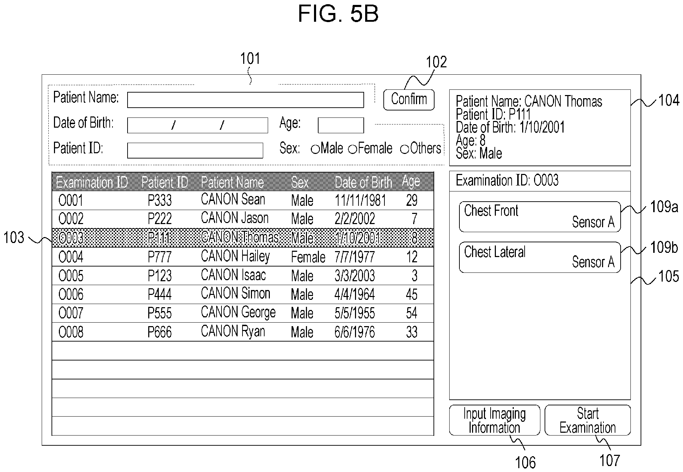

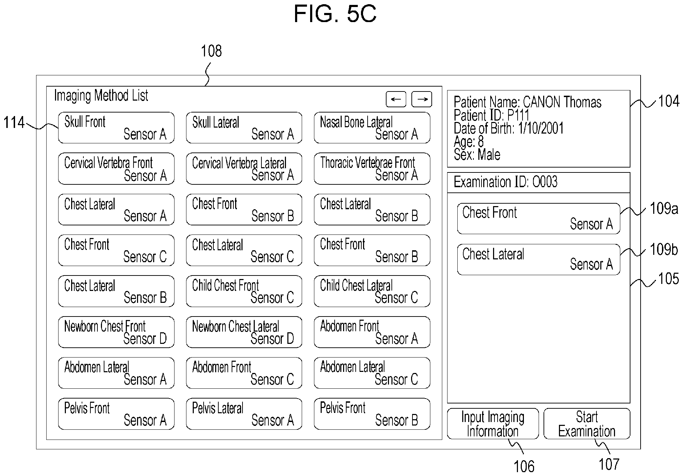

In response to selection of an examination from the requested examination list 103, the patient information display area 104 displays patient information (such as the patient ID, the patient name, and the date of birth) of a patient corresponding to the selected examination as illustrated in Fig. 5B. The imaging information display area 105 displays an examination ID, and displays imaging information corresponding to the examination ID immediately below the examination ID. As described above, the imaging information is received from the RIS 12. In the example of Fig. 5B, imaging method buttons 109 (a chest front button 109a and a chest lateral button 109b) corresponding to the imaging information are arranged. In response to pressing of the imaging information input button 106, an imaging information input area 108 is displayed as illustrated in Fig. 5C. This may allow further addition of an imaging method. In the example of Fig. 5C, the imaging information input area 108 displays a plurality of imaging method selection buttons 114. An imaging method can be added by selecting one of these imaging method selection buttons 114. The added imaging method is displayed in the imaging information display area 105 along with the chest front button 109a and the chest lateral button 109b. Each imaging method is associated with an imaging method ID.

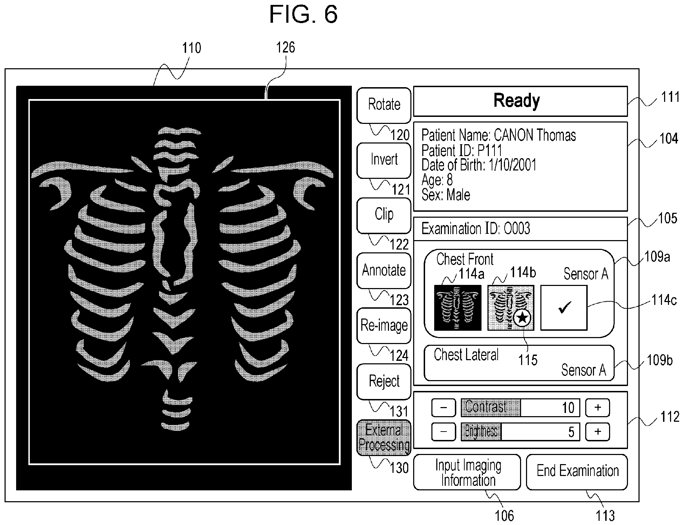

After confirming the patient information and the imaging information, an operator presses the examination start button 107. In this way, an examination to be performed is determined. In response to pressing of the examination start button 107, the radiographing apparatus 1 displays an imaging screen illustrated in Fig. 6 on the display unit 2. The imaging screen is a screen used during imaging.

The imaging screen includes basically the same display areas as the new examination input screen described with reference to Figs. 5A to 5C. Display areas newly added include an image display area 110, a message area 111, an image processing setting area 112, and an examination end button 113 as illustrated in Fig. 6.

When the imaging screen is displayed, the imaging method button 109a at a topmost portion in the imaging information display area 105 is in a selected state by default. Thus, the control unit 5 of the radiographing apparatus 1 transmits imaging conditions (such as a tube voltage, a tube current, and an irradiation time) set for the imaging method button 109a (e.g., imaging method) to the radiation-generator control unit 4. The control unit 5 then controls the radiation detector 7 in accordance with the imaging conditions to prepare for imaging.

After finishing the preparation, the radiographing apparatus 1 enters a ready-to-image state. At this time, the message area 111 displays a message "Ready" indicating the ready-to-image state.

Subsequently, an operator confirms the imaging method, performs imaging settings, and performs positioning of the patient. After finishing a series of preparations for imaging, the operator confirms the ready-to-image state with reference to the message area 111 and then presses a radiation irradiation switch (not illustrated). The radiographing apparatus 1 then causes the radiation generator 8 to irradiate a subject (e.g., a specific portion of the patient) with radiation, and causes the radiation detector 7 to detect radiation transmitted through the subject. In this way, a radiographic image is captured.

After finishing the imaging, the control unit 5 of the radiographing apparatus 1 obtains a captured image from the radiation detector 7 and performs image processing on the obtained captured image, based on a predetermined image processing condition. The predetermined image processing condition is defined in advance for the imaging method.

After finishing the image processing, the radiographing apparatus 1 displays the captured image on which the image processing has been performed in the image display area 110. The radiographing apparatus 1 also creates a thumbnail 114a in the imaging method button 109.

In a case where the operator desires to change contrast or the like of the captured image, the operator operates a button such as a contrast button or a brightness button provided in the image processing setting area 112.

Likewise, in a case where the operator desires to change a clipping area of the output image, the operator operates a clip button 122, a clipping frame 126, and the like to designate a desired clipping area. In a case where the operator assigns a character string serving as diagnostic information, the operator operates an annotate button 123 or the like to superimpose the character string on the image. In a case where the orientation of the image is not suitable for diagnosis, the operator uses a rotate button 120, an invert button 121, or the like to perform geometric transformation. As described above, the operator can perform additional image editing on the captured image displayed in the image display area 110.

In a case where the imaging has failed because of a body movement or the like of the subject, the operator operates a re-image button 124 to perform re-imaging. When re-imaging is performed, a failed image (e.g., a rejected image) is treated as being rejected. In a case where re-imaging is not necessary, the failed image is treated as being rejected without performing re-imaging in response to an operation on a reject button 131.

When the operator desires to use image processing in the external processing apparatus 17, the operator presses an external processing button 130. In the imaging method, whether the external processing is enabled, and in a case where the external processing is enabled, a destination of a request for the external processing and content of the external processing are set in advance. Alternatively, the control unit 5 may set whether to enable transmission of a medical image to the external processing apparatus 17. In a case where a plurality of external processing apparatuses 17 are present, whether to enable transmission of a medical image may be set for each of the plurality of external processing apparatuses 17, or whether to enable the transmission may be set collectively. For example, when an image corresponding to the chest front button 109a is displayed in the image display area 110, the external processing button 130 is enabled in accordance with the setting as to whether to enable the external processing of the imaging method corresponding to the chest front button 109a.

In response to pressing of the external processing button 130, the radiographic image displayed in the image display area 110 is transmitted to the external processing apparatus 17. That is, an image serving as a processing target can be transmitted to the external processing apparatus 17 when transmission of a medical image is enabled. In a case where a plurality of external processing apparatuses 17 are present, the medical image is transmitted to the external processing apparatus 17 for which a setting of making a processing request is set in advance among the plurality of external processing apparatuses 17. The external processing apparatus 17 serving as the transmission destination is determined in accordance with the request destination set in the imaging method corresponding to the chest front button 109a. The radiographic image to be transmitted is accompanied with an image ID for identifying the radiographic image and information indicating the content of processing to be performed by the external processing apparatus 17. That is, information on the content of processing to be performed on the medical image is transmitted along with the medical image, and the external processing apparatus 17 performs processing based on the transmitted processing content. In a configuration in which image processing in the external processing apparatus 17 is always used, a captured image may be transmitted in response to generation of the captured image through imaging without pressing of the external processing button 130. That is, an original image serving as a processing target in the external processing apparatus 17 may be transmitted to the external processing apparatus 17 at a generation timing of the original image, or the image may be transmitted to the external processing apparatus 17 in response to the operator pressing the external processing button 130. Control is performed such that an indication, for example, a thumbnail 114c, indicating that the processing request has been made is provided in the imaging method button 109.

The examination order received from the RIS 12 includes imaging information. The HIS 11 and the RIS 12 may request image generation using external processing by designating an imaging method in which the setting for enabling external processing is made.

In response to receipt of a processed image from the external processing apparatus 17, the processed image is associated with the captured image by using a captured image ID, of the processing-target original, accompanying the processed image. That is, the examination management unit 24 can search the storage unit 23 for the original image for the processing by using the accompanying information of the processed image as association information for associating the original image with the processed image, and associate the extracted original image with the processed image. Alternatively, information on the processed image such as the captured image ID may be received by communication different from reception of the processed image. That is, the examination management unit 24 can search the storage unit 23 for the original image for the processing by using the association information of the processed image transmitted by communication different from that of the processed image, and associate the extracted original image with the processed image. The associated processed image is displayed as a thumbnail in the same imaging method button 109a as the captured image. That is, the control unit 5 corresponds to an example of display control means configured to display a thumbnail of an original image and a thumbnail of a processed image on a display unit based on association. The control unit 5 also corresponds to an example of display control means configured to display the thumbnail of the original image and the thumbnail of the processed image over an object indicating an imaging protocol used during capturing of the original image in a superimposed manner. A style presenting the thumbnail 114c at the time of a processing request to indicate that the processing request has been made is changed to a style displaying a reduced-size image such as a thumbnail 114b. Over the thumbnail 114b, an icon is displayed which indicates that the corresponding image is a processed image generated by the external processing apparatus 17 and indicates the external processing apparatus 17 that has performed the processing and content of the processing. That is, the thumbnail of the processed image can be displayed in a different display style from the thumbnail of the original image to be subjected to processing in the external processing apparatus 17 to make the processed image identifiable as being an image that has been subjected to the processing. In response to pressing of the thumbnail, the processed image is displayed in the image display area 110. In a case where reprocessing in the radiographing apparatus 1 is not necessary for the processed image, operations for manipulating and processing the image through the rotate button 120, the invert button 121, the clip button 122, the annotate button 123, and the image processing setting area 112 can be disabled. Alternatively, in a case where an image editing instruction is received for the processed image, a warning indicating that processing of the image is restricted may be displayed.

In response to designation of the imaging method button 109b for the next imaging, the control unit 5 controls the radiation detector 7 in accordance with the imaging conditions to prepare for imaging. In response to designation of the imaging method button 109b and emission of radiation, the control unit 5 may regard confirmation of the image captured with the imaging method button 109a as being completed.

The operator repeats the procedure described above to perform imaging with all the imaging methods in the imaging information display area 105. After finishing all the imaging, the operator presses the examination end button 113. In this way, an examination series ends. The control unit 5 in the radiographing apparatus 1 assigns the examination information, the imaging conditions, and the like as accompanying information, and the radiographing apparatus 1 then outputs the image object to, for example, the PACS 13, the printer 14, the ROM of the radiographing apparatus 1, or the like. For the processed image, for example, as in the imaging method table in Fig. 3, whether to output the processed image to an image management server is settable in advance for each imaging method. The radiographing apparatus 1 outputs the processed image obtained with the imaging method for which output is enabled. That is, the radiographing apparatus 1 outputs the processed image to the image management server, based on the setting as to whether to enable the output of the processed image to the image management server. The radiographing apparatus 1 transmits examination execution information reporting the end of the examination to the HIS 11. An identifier of the processed image received by the end of the examination is included in the examination execution information as an object in the examination. An image (rejected image) that is treated as being rejected by an operation on the re-image button 124 or the reject button 131 is excluded from the output target.

The radiographing apparatus 1 displays the new examination input screen again.

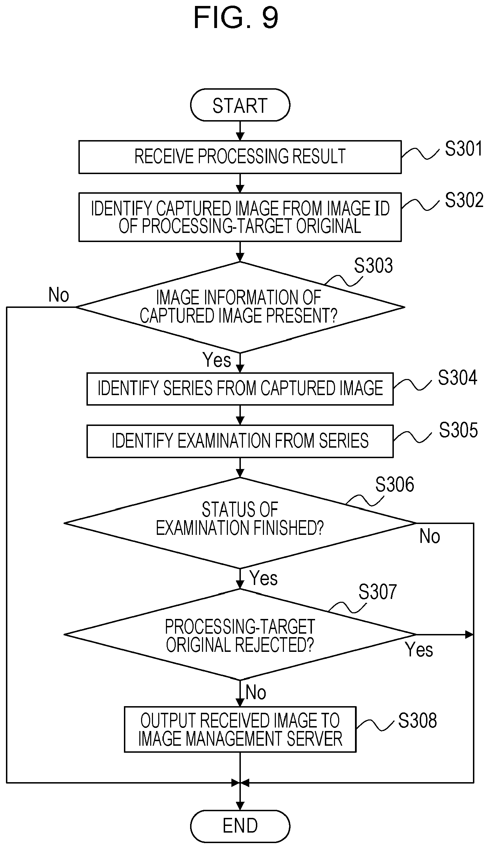

The radiographing apparatus 1 may receive a processed image from the external processing apparatus 17 after the end of the examination. Fig. 9 is a flowchart illustrating an example of a procedure of an output process of outputting an image to the image management server, as a medical information processing method performed by the medical information processing apparatus according to the present embodiment. In step S301, the radiographing apparatus 1 receives an image (e.g., processed image) generated by the external processing apparatus 17. The received processed image is accompanied with the image ID of the processing-target original (e.g., captured image). In step S302, the radiographing apparatus 1 searches the image table for image information of the captured image by using the image ID as a key. In a case where the image information is not extracted ("No" in step S303), the process of the flowchart of Fig. 9 ends. In a case where the image information is extracted ("Yes" in step S303), the process proceeds to step S304. In step S304, the radiographing apparatus 1 identifies a series from the captured image. The radiographing apparatus 1 identifies a series ID from the image information of the captured image retrieved in step S302.

In step S305, the radiographing apparatus 1 identifies an examination from the series. The radiographing apparatus 1 identifies an examination ID from the series ID identified in step S304. In step S306, the radiographing apparatus 1 determines whether the status of the examination is "finished". The radiographing apparatus 1 obtains the status of the examination from the examination ID identified in step S305, and determines whether the status is "finished". In a case where it is determined that the status is "finished" ("Yes" in step S306), the process proceeds to step S307. In a case where it is determined that the status is not "finished" ("No" in step S306), the process of the flowchart of Fig. 9 ends. In this case, a series of images including the image received in step S301 is output to the image management server at a finished timing of the examination. In step S307, the radiographing apparatus 1 determines whether the processing-target original image is a rejected image. In a case where it is determined that the processing-target original image is not a rejected image ("No" in step S307), the radiographing apparatus 1 outputs, in step S308, the image received in step S301 to the image management server. In a case where it is determined that the processing-target original image is a rejected image ("Yes" in step S307), the process of the flowchart in Fig. 9 ends.

Alternatively, the radiographing apparatus 1 can, in a case where an examination including capturing of the original image is displayed on the display unit 2 when the processed image is obtained, output the processed image to the image management server in response to an output instruction, and in a case where the examination is not displayed on the display unit 2 when the processed image is obtained, output the processed image to the image management server at an obtained timing of the processed image.

### Processed Image Registration Process

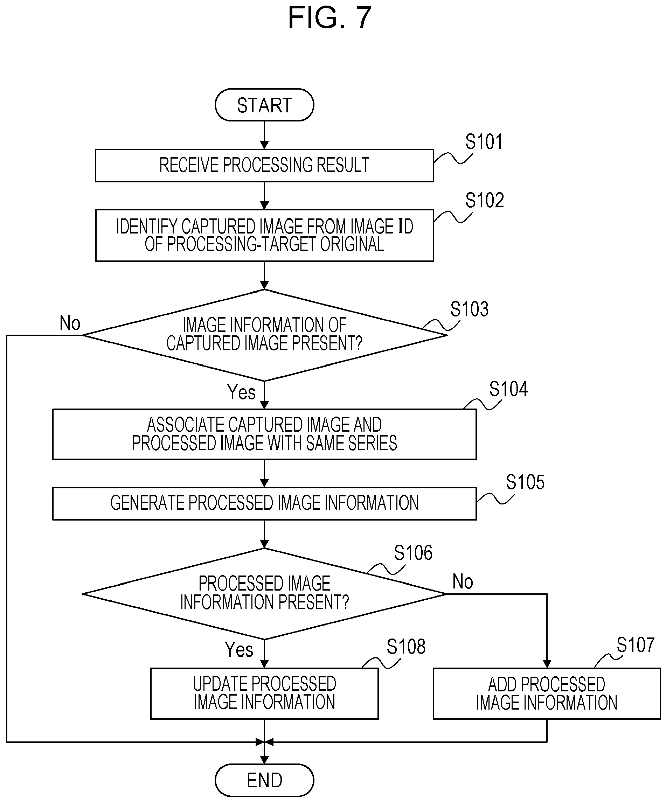

Fig. 7 is a flowchart illustrating an example of a procedure of a processed image registration process, as a medical information processing method performed by the medical information processing apparatus according to the present embodiment. Description will be given with reference to the imaging screen illustrated in Fig. 6. First, in response to an operator pressing the external processing button 130, the radiographing apparatus 1 outputs an image object being displayed to the external processing apparatus 17. The external processing apparatus 17 finishes the processing. In step S101, the radiographing apparatus 1 receives a generated processed image. The received processed image is accompanied with an image ID of the corresponding processing-target original (e.g., captured image). In step S102, the radiographing apparatus 1 searches the image table for image information of the captured image by using the image ID as a key. In a case where the image information is not extracted ("No" in step S103), the process of the flowchart of Fig. 7 ends. In a case where the image information is extracted ("Yes" in step S103), the process proceeds to step S104. In step S104, the radiographing apparatus 1 associates the processed image with the same series as the captured image. The radiographing apparatus 1 uses the series ID in the image information of the captured image retrieved in step S102, as the series ID of the processed image. In step S105, the radiographing apparatus 1 generates processed image information. In a case where the captured image retrieved in step S102 is rejected, the processed image information is generated to indicate that the processed image is also rejected. The processed image received in step S101 is accompanied also with an ID for identifying the processed image. In step S106, the radiographing apparatus 1 searches the image table by using the ID for identifying the processed image and determines whether information of the same processed image is stored. In a case where it is determined that information of the same processed image is present ("Yes" in step S106), the radiographing apparatus 1 updates, in step S108, the information with the information generated in step S105. In a case where it is determined that the information of the same processed image is absent ("No" in step S106), the radiographing apparatus 1 adds, in step S107, the information generated in step S105. That is, the processing in step S106 to step S108 corresponds to processing of, in a case where association information of a first processed image already associated with an original image is determined to be identical to association information of a second processed image newly obtained, updating the association information of the first processed image with the association information of the second processed image, and in a case where the association information of the first processed image is determined to be different from the association information of the second processed image, newly adding the association information of the second processed image.

### Processed Image Display Process

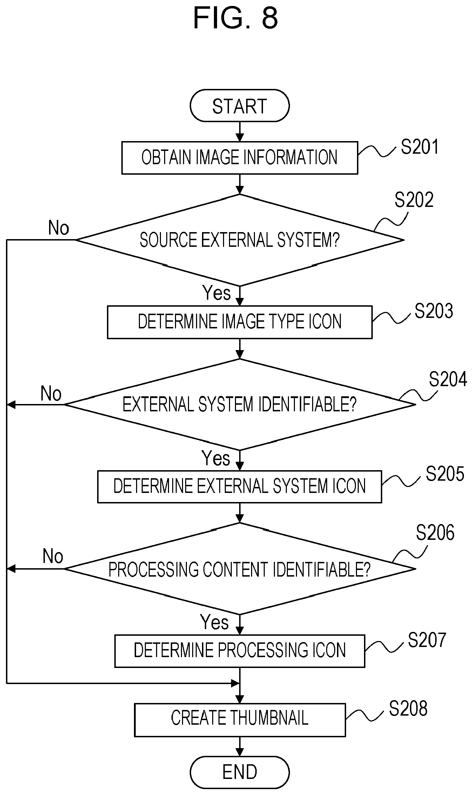

Fig. 8 is a flowchart illustrating an example of a procedure of a processed image display process on a screen, as a medical information processing method performed by the medical information processing apparatus according to the present embodiment. Description will be given with reference to the imaging screen illustrated in Fig. 6.

First, in step S201, the radiographing apparatus 1 obtains target image information from the image table. The radiographing apparatus 1 refers to a source attribute of the image information. In a case where the source is not the external processing apparatus 17 (e.g., an external system) ("No" in step S202), the process proceeds to step S208. In a case where the source is the external processing apparatus 17 (e.g., external system) ("Yes" in step S202), the process proceeds to subsequent external processing icon determination processing. First, in step S203, an image type icon is determined. Since it is determined in step S202 that the source is the external processing apparatus 17, an icon indicating that the image type is the external processing apparatus 17 is determined.

In a case where which external processing apparatus 17 (e.g., external system) is not identifiable with reference to the source information of the image information obtained in step S201 ("No" in step S204), the process proceeds to step S208. In a case where the external processing apparatus 17 (external system) is identifiable ("Yes" in step S204), the process proceeds to step S205, in which an external processing apparatus icon (e.g., external system icon) is determined. The external processing apparatus icon to be used may be an icon representing a characteristic or the like of the external processing apparatus 17.

In a case where the processing content is not identifiable with reference to the processing content of the image information obtained in step S201 ("No" in step S206), the process proceeds to step S208. In a case where the processing content is identifiable ("Yes" in step S206), a processing icon is determined. The processing icon to be used may be an icon representing the content of processing that has been performed.

After the completion of the external processing icon determination processing described above, the process proceeds to step S208, in which a thumbnail is created and displayed on the screen. For example, an icon 115 is displayed over a thumbnail image such as the thumbnail 114b in the imaging method button 109a in a superimposed manner. Note that the display style of the icon 115 is not limited to the superimposed display described above and may be composite display or parallel display. The image type icon, the external processing apparatus icon, and the processing icon have been described separately. However, these icons may be combined into a single icon. Note that the display indicating the above information may be text data or the like, or may be a thumbnail-surrounding frame line that emphasizes the thumbnail of the processed image as compared to the thumbnail of the original image. That is, any object may be used which enables the thumbnail of the processed image and the thumbnail of the original image to be distinguished from each other. The text data may represent a difference in information by changing a font, a character size, or a character color. That is, an object indicating that the processed image is an image that has been subjected to the processing in the external processing apparatus 17 can be displayed, and the thumbnail of the processed image and the object indicating that the processed image is an image that has been subjected to the processing in the external processing apparatus 17 can be displayed in any display style of superimposed display, parallel display, and composite display. In addition, an object indicating the external processing apparatus 17 that has performed the processing for the processed image can be displayed based on the information on the external processing apparatus 17, and the thumbnail of the processed image and the object indicating the external processing apparatus 17 that has performed the processing for the processed image can be displayed in any display style of superimposed display, parallel display, and composite display. An object indicating content of the processing that has been performed for the processed image can be displayed, and the thumbnail of the processed image and the object indicating the content of the processing that has been performed for the processed image can be displayed in any display style of superimposed display, parallel display, and composite display. An object indicating that the original image is an image to be subjected to the processing in the external processing apparatus 17 may be displayed, and the thumbnail of the original image and the object may be displayed in any display style of superimposed display, parallel display, and composite display. That is, the thumbnail of the processed image may be displayed in a different display style from the thumbnail of the original image to make the processed image identifiable as being an image that has been subjected to the processing in the external processing apparatus 17.

The configuration described above allows the captured image and the processed image to be distinguished from each other. Even in a case where the processing which the operator is permitted to perform differs from image to image, the efficiency of the operation can be increased since the captured image and the processed image can be distinguished from each other. Displaying the object indicating the external processing apparatus 17 that has performed the processing for the processed image on the display unit 2 can make it easier to grasp which of the external processing apparatuses 17 has performed the processing for the processed image without reference to the accompanying information. Displaying the object indicating the content of the processing that has been performed for the processed image on the display unit 2 can make it easier to grasp the content of the processing that has been performed for the processed image without reference to the accompanying information. The captured image and the processed image are automatically associated with each other, which omits the necessity of association by a manual operation and thus can increase the efficiency of the operation.

For example, the processed image received from an external image processing apparatus is transmitted to the image management server together with the processing-target original image upon the end of the examination, so that a doctor can refer to the processed image. However, a trigger for transmission may be missed unless the examination is finished after a complete set of the processed images is obtained. Consequently, the complete set of the processed images may not be in the image management server. On the other hand, the efficiency of the examination decreases in a case where the end of the examination is waited until the complete set of the processed images is obtained. Accordingly, the present disclosure enables a processed image to be transmitted to an image management server irrespective of the progress of an examination. A medical information processing apparatus according to an embodiment of the present disclosure includes transmission means configured to transmit an original image to an external processing apparatus, the original image being a medical image captured by an imaging apparatus and serving as a processing target in the external processing apparatus. The medical information processing apparatus according to the embodiment of the present disclosure also includes obtaining means configured to obtain a processing result generated from the original image through processing performed in the external processing apparatus. The medical information processing apparatus according to the embodiment of the present disclosure further includes output means configured to output the processing result to the image management server, based on a setting as to whether to enable output of the processing result to the image management server. In the medical information processing apparatus according to the embodiment of the present disclosure, in a case where an examination including capturing of the original image is not ended by an obtained timing of the processing result, the output means outputs the processing result to the image management server in response to an end of the examination, and in a case where the examination is ended by the obtained timing of the processing result, the output means outputs the processing result to the image management server at the obtained timing of the processing result. The present disclosure enables a processed image to be transmitted to an image management server irrespective of the progress of an examination.

### Other Embodiments

Embodiment(s) of the present invention can also be realized by a computer of a system or apparatus that reads out and executes computer executable instructions (e.g., one or more programs) recorded on a storage medium (which may also be referred to more fully as a 'non-transitory computer-readable storage medium') to perform the functions of one or more of the above-described embodiment(s) and/or that includes one or more circuits (e.g., application specific integrated circuit (ASIC)) for performing the functions of one or more of the above-described embodiment(s), and by a method performed by the computer of the system or apparatus by, for example, reading out and executing the computer executable instructions from the storage medium to perform the functions of one or more of the above-described embodiment(s) and/or controlling the one or more circuits to perform the functions of one or more of the above-described embodiment(s). The computer may comprise one or more processors (e.g., central processing unit (CPU), micro processing unit (MPU)) and may include a network of separate computers or separate processors to read out and execute the computer executable instructions. The computer executable instructions may be provided to the computer, for example, from a network or the storage medium. The storage medium may include, for example, one or more of a hard disk, a random-access memory (RAM), a read only memory (ROM), a storage of distributed computing systems, an optical disk (such as a compact disc (CD), digital versatile disc (DVD), or Blu-ray Disc (BD)^{™}), a flash memory device, a memory card, and the like.

While the present invention has been described with reference to embodiments, it is to be understood that the invention is not limited to the disclosed embodiments. The scope of the following claims is to encompass all such modifications and equivalent structures and functions.

## Claims

1. A medical information processing apparatus comprising:
an obtaining means (5, 27) configured to obtain a processing result of an original image that is a medical image, the processing result being obtained through processing performed in an external processing apparatus (17); and
an output means (5, 27) configured to output the processing result to an image management server, based on a setting as to whether to enable output of the processing result to the image management server,
wherein the output means is configured, in a case where an examination including capturing of the original image is not ended by an obtained timing of the processing result, to output the processing result to the image management server in response to an end of the examination, and
wherein the output means is configured, in a case where the examination is ended by the obtained timing of the processing result, to output the processing result to the image management server at the obtained timing of the processing result.

2. The medical information processing apparatus according to Claim 1, wherein the processing result includes an image generated from the original image as a result of the processing performed by the external processing apparatus (17).

3. The medical information processing apparatus according to Claim 1 or Claim 2, wherein the obtaining means (5) is configured to treat the processing result as being rejected in a case where the original image is determined to be a rejected image.

4. The medical information processing apparatus according to any one of Claims 1 to 3, wherein the output means (5, 27) is configured to not output the processing result to the image management server in a case where the original image is determined to be a rejected image.

5. The medical information processing apparatus according to any one of Claims 1 to 4, further comprising:
a display control means (5) configured to display a thumbnail of the original image and a thumbnail of a processed image included in the processing result on a display unit (2),
wherein the display control means (5) is configured to display the thumbnail of the processed image in a different display style from the thumbnail of the original image to make the processed image identifiable as an image that has been subjected to the processing in the external processing apparatus (17).

6. The medical information processing apparatus according to any one of Claims 1 to 4, further comprising:
a display control means (5) configured to display a thumbnail of a processed image included in the processing result on a display unit (2), wherein the display control means (5) is configured to display an object indicating that the processed image is an image that has been subjected to the processing in the external processing apparatus (17), and display the thumbnail of the processed image and the object in at least one of a superimposed display style, a parallel display style, and a composite display style.

7. The medical information processing apparatus according to any one of Claims 1 to 4, further comprising:
a display control means (5) configured to display a thumbnail of the original image on a display unit (2), wherein the display control means (5) is configured to display an object indicating that the original image is an image to be subjected to the processing in the external processing apparatus (17), and display the thumbnail of the original image and the object in at least one of a superimposed display style, a parallel display style, and a composite display style.

8. The medical information processing apparatus according to any one of Claims 1 to 7, further comprising:
an accepting means (5) configured to accept an image editing instruction for a medical image from an operator, wherein the accepting means (5) is configured to restrict an image editing instruction for a processed image included in the processing result or request display control means (5) to display a warning in response to accepting an image editing instruction for the processed image.

9. The medical information processing apparatus according to any one of Claims 1 to 8, further comprising:
a transmission means (27) configured to transmit the original image to the external processing apparatus (17); and
a setting means (5) configured to make a setting as to whether to enable transmission of a medical image to the external processing apparatus (17), wherein the transmission means (27) is configured to transmit the original image to the external processing apparatus (17) in a case where transmission of a medical image is enabled by the setting means (5).

10. The medical information processing apparatus according to any one of Claims 1 to 9, wherein the output means (5, 27) is configured, in a case where the examination including capturing of the original image is displayed on a display unit (2) when a processed image included in the processing result is obtained by the obtaining means (5, 27), to output the processed image to the image management server in response to an output instruction, and
wherein the output means (5, 27) is configured, in a case where the examination is not displayed on the display unit (2) when the processed image is obtained by the obtaining means (5, 27), to output the processed image to the image management server at an obtained timing of the processed image by the obtaining means (5, 27).

11. A medical information processing method comprising:
obtaining (S301) a processing result of an original image that is a medical image, the processing result being obtained through processing performed in an external processing apparatus (17); and
outputting (S308) the processing result to an image management server, based on a setting as to whether to enable output of the processing result to the image management server,
wherein in a case where an examination including capturing of the original image is not ended by an obtained timing of the processing result, the processing result is output to the image management server in response to an end of the examination, and
wherein in a case where the examination is ended by the obtained timing of the processing result, the processing result is output to the image management server at the obtained timing of the processing result.

12. A computer program for causing a computer to execute the medical information processing method according to Claim 11.
